(19) Europäisches Patentamt

European Patent Office

Office européen des brevets

(11) Publication number: **0 174 195 B1**

# EUROPEAN PATENT SPECIFICATION

(45) Date of publication of patent specification: **07.08.91**  (51) Int. Cl.⁵: **G01N 33/545**, G01N 33/532

(21) Application number: **85306302.2**

(22) Date of filing: **05.09.85**

The file contains technical information submitted after the application was filed and not included in this specification

(54) **Diagnostic test methods.**

(30) Priority: **06.09.84 GB 8422512**
**10.07.85 GB 8517477**

(43) Date of publication of application:
**12.03.86 Bulletin 86/11**

(45) Publication of the grant of the patent:
**07.08.91 Bulletin 91/32**

(84) Designated Contracting States:
**AT BE CH DE FR GB IT LI LU NL SE**

(56) References cited:
**EP-A- 0 032 270**
**EP-A- 0 070 527**

(73) Proprietor: **THE WELLCOME FOUNDATION LIMITED**
**Unicorn House 160 Euston Road**
**London NW1 2BP(GB)**

(72) Inventor: **Hadfield, Susan Gaye**
**Langley Court**
**Beckenham Kent(GB)**
Inventor: **Norrington, Franklin Edward Anthony**
**Langley Court**
**Beckenham Kent(GB)**

## Description

The present invention relates to a method of testing for the presence of a ligand and to a reagent suitable for use therein.

Diagnostic test methods based upon the agglutination of immunogens and antibodies, wherein either the immunogen or the antibody is attached to a solid phase, are well known in the field of immunodiagnostic reagents. For example, US Patent 3088875 describes a technique wherein plastics microspheres coated with antigen are mixed with a test sample such that when the sample contains antibodies to the antigen, the antibodies attach themselves to the antigen thereby causing visible agglutination or aggregation of the microspheres.

Coloured solid phases or particles have been used to aid visualisation of the agglutination process. For example, there are marketed test kits for the grouping of Beta Haemolytic Streptococci which include reagents in which the solid phase is a suspension of killed red-dyed or blue-dyed Staphylococcus aureus cells. There is also marketed a test kit containing four separate reagents, each of which contains a specific, different colour of latex particle, for the respective detection and grouping of streptococci A,B,C and G; by assigning a specific colour to each test reagent, any confusion which could be caused by eg incorrect labelling is avoided.

US Patent 4419453 describes a latex agglutination test in which the test reagent comprises antigen-or antibody-coated latex particles of one colour and a water-soluble, non-latex polymer particle absorbing dye of a different colour; when agglutination takes place, the contrast between the colour of the agglutinate and the background colour of the solution assists visualisation.

EP-A-0 032 270 discloses an immunoassay procedure for the determination of an "immunochemically reactive component" utilizing a reagent obtained by coupling such a component to particles of an aqueous dispersion of a hydrophobic dye or pigment, the target "immunochemically reactive component" being a hapten, antigen or antibody. This text further teaches the possibility of determining two or more such components simultaneously by using in the said manner chromophores that are clearly distinguishable spectrophotometrically. Each chromophore-labelled component species in such a "polyvalent" reagent is thus necessarily immunochemically reactive with a corresponding target component (hapten, antigen or antibody), Example 7 illustrating the simultaneous determination of human chorionic gonadotropin (HCG) and human placental lactogen (HPL) using a divalent reagent comprising a mixture of individually labelled anti-HCG and anti-HPL.

A diagnostic technique such as a latex agglutination test is generally used to reinforce an initial diagnosis based upon the clinical symptoms exhibited by a patient suffering from a particular disease. In cases where a disease is characterised by very distinctive symptoms, confirmation of the presence of a causative agent (eg bacterium or virus) could involve relatively few tests. However, with diseases in which the symptoms could be ascribed to any one of a large number of causative agents, it will be apparent that much time and effort could be expended in performing a test against each of the possible causative agents and that the size of sample required from the patient could be considerable. This latter aspect could be a serious problem in instances (eg when the test liquid is neonatal cerebrospinal fluid (CSF)) where it is possible to take only small samples of biological material from the patient.

There thus exists a need for a technique which is rapid, safe, simple and broadly applicable and which can significantly reduce the volume of biological material required to be taken from a patient.

In conventional latex agglutination techniques, it is usual to employ a separate control reagent which is a suspension of latex particles coated with the immunoglobulin fraction of an animal that has not been inoculated with the antigen under test, or a monoclonal antibody of the same class as that used on the test latex but having a different specificity. Agglutination of the control latex in the presence of a test sample indicates a non-specific interaction, due for example to the presence of interfering substances such as Rheumatoid Factor (RF) or Protein A (found on most S.aureus bacteria) and Protein A-like substances (found on some streptococci).

The drawback to using such a control reagent is that at least one additional aliquot of biological test liquid is required for each test or series of tests, this additional aliquot subsequently yielding no useful information as to the identity of the infecting agent.

There has now been discovered a technique in which the disadvantageous requirement for testing with a separate control reagent is overcome.

In one aspect the present invention thus provides a method of testing for the presence of a ligand in an aqueous liquid medium comprising the steps of:

A. mixing a sample of the said medium with a reagent which comprises

particles of a first colour having attached thereto antibody bindable to the said ligand, in admixture with

2

particles of a second colour having attached thereto immunoglobulin from a non-inoculated animal, whereby non-specific interaction between the said sample and the said reagent is indicated by agglutination of both the said particles of the first colour and the said particles of the second colour and the presence of the said ligand in the said sample, free from said non-specific interaction, is indicated by agglutination of the said particles of the first colour alone;

B. observing whether or not agglutination occurs; and

C. determining the colour of the aggulutinate.

The present invention also provides a test reagent for the presence of a ligand in a sample of an aqueous liquid medium, said reagent comprising

particles of a first colour having attached thereto antibody bindable to the said ligand, in admixture with particles of a second colour having attached thereto immunoglobulin from a non-inoculated animal, whereby non-specific interaction between the said sample and the said reagent is indicated by agglutination of both the said particles of the first colour and the said particles of the second colour and the presence of the said ligand in the said sample, free from said non-specific interaction, is indicated by agglutination of the said particles of the first colour alone.

For example, in a typical test procedure, the test sample is mixed with a reagent comprising blue particles having attached thereto antibody bindable to the ligand to be detected and red particles having attached thereto immunoglobulin from a non-inoculated animal. If the suspected ligand is present and there is no non-specific interaction, a blue agglutinate will be formed against a red background. If non-specific interaction does occur, both red and blue particles will agglutinate giving rise to clumps of a purplish colour.

It will be apparent to the skilled man that although red and blue particles are described by way of example, any two (contrasting) colours could be used in place thereof.

The term ligand, as used herein, includes antigens, haptens, monoclonal and polyclonal antibodies and other substances capable of being bound by antibodies.

The substance tested may be a biological material, such as a body fluid taken from an animal, human or otherwise, or it may be any other material in which a ligand may be found including for example culture broths, suspensions from liquid or solid growth media, culture supernatants, tissue culture supernatants, enzyme-or chemically-extracted material from bacteria or viruses (eg Lancefield extracts for serological grouping of Streptococci), foodstuffs and environmental samples (eg water samples from the public supply).

Biological materials which can be taken from animals include cerebrospinal fluid, blood, urine, sputum, tissue extracts, sweat, tears, secretions, faeces, mucus and synovial fluid.

The above list is not intended to be exhaustive and the skilled man will appreciate that other types of biological material may be taken and tested by the method of the present invention.

The coloured particles are preferably of microscopic size. Suitable materials which are coloured, or which can be dyed, include non-viable bacterial cells, alginate particles, Sepharose beads, silica, alumina, erythrocytes and polymer latexes such as polystyrene latex, styrene-glycidyl methacrylate latex and other polymer latexes such those described in US Patent 4419453. Coloured particles may be prepared or dyed according to standard methods, see for example US Patent 4419453 and German Patent Application DT-3000-483, or they may be purchased from an appropriate source. Particularly suitable colours include red, yellow, blue, green, black, cyan, magenta and white.

The agglutinate is preferably visible to the naked eye. Visualisation of the agglutinate is assisted by the colour contrast thereof with the background provided by the non-agglutinated particles.

The antibody or immunoglobulin may be attached to the particles by adsorption, by chemical coupling, by incorporation into the particles or by any other method known in the art.

Adsorption of the antibody of immunoglobulin onto the particles is typically achieved by incubation of the particles with a suitably buffered solution of the former. Chemical coupling can be achieved by for example the method described in US Patent 4436826 wherein a carbodiimide coupling reagent is employed; a similar coupling process is described in US Patent 4140662.

The method and reagent of the present invention are especially useful for the detection of bacterial, viral or parasitic infections and the identification of antigen or antibody in biological fluids. They are particularly useful in the analysis of cerebrospinal fluid (eg neonatal cerebrospinal fluid) for such species as Haemophilus influenzae, Neisseria meningitidis and Streptococcus pneumoniae.

The method and reagent are also useful for the identification of serologically distinct strains eg Streptococcal serogroups A,B,C,D,F and G, Salmonella O or H antigens and Meningococci serogroups A,B,C,Y,29E and Z.

It is desirable in certain instances to pre-treat the medium before testing; such methods of pre-treatment include treatment with acids, enzyme extraction, filtering, centrifuging, diluting, concentrating and heating. By heating, for example, it is possible to deactivate, or significantly reduce the activity of, the abovementioned interfering substances.

Items which can be included in kits suitable for use in the method of the present invention include spotting cards, mixing sticks, a positive control antigen for the antigen being tested, negative control "antigen" (eg saline solution) and a set of instructions for use of the kit.

The present invention will now be illustrated by means of examples. The examples should not be construed as imposing a limitation on the scope of the invention.

REFERENCE EXAMPLE 1 Preparation of Sensitised Latex

I Preparation of antibody.

Immunoglobulin G was obtained partially purified from immune rabbit sera by treatment with octanoic acid (BDH Chemicals Ltd.) using the method of Steinbuch and Audran (Arch. Biochem. and Biophys, 134, 279-284, 1969).

II. Binding of antibody to coloured latex.

To 5.0mg of coloured latex (Estapor(trade name), K58, 0.2 $\mu$m, polystyrene, black, red, blue, yellow or green, Rhone-Poulenc. EP-A-0 085 016 was added 600$\mu$g. of antibody in 1ml. of glycine saline pH 8.2 (0.1M glycine in 0.85% NaCl, pH adjusted to 8.2 with NaOH). Latex and antibody were heated at 56°C for 30 mins. After cooling to room temperature, bovine albumin (Miles Laboratories Ltd.) was added to give a 1%(w/v) concentration.

III. Preparation of polyvalent latex.

Three different coloured latexes, each one sensitised with antibody of a different specificity, eg. red latex coated with antibody to Salmonella serogroup A, blue latex coated with antibody to serogroup B and green latex coated with antibody to serogroup C, were mixed together in equal proportions. The resultant mixture was brown in colour.

REFERENCE EXAMPLE 2 Use of the polyvalent Latex.

Latex agglutination tests were performed on white cards (Syfacard -R(trade name), Wellcome Diagnostics Ltd.). Equal volumes of the test sample and the polyvalent latex (usually 20$\mu$l) were mixed together on a circle, diameter 2cm., on the white card. The card was rocked for 3 mins after which the mixture was examined for agglutination and the colour of any agglutinate identified eg. red, blue or green; at the same time the colour of the unagglutinated latex changed from brown to a combination of the two colours of the particles remaining unagglutinated in suspension.

(a) Bacterial colony identification.

A single colony of bacteria was removed from a solid growth medium and emulsified in 200$\mu$l of 0.85% saline. The bacterial suspensions were mixed with the polyvalent latex as described above.

| ANTIGEN | | RESULT |
|---|---|---|
| 1. | Saline only | Brown, homogeneous |
| 2. | Salmonella Serogroup A eg. S.paratyphi A | Red agglutinate, turquoise background |
| 3. | Salmonella Serogroup B eg. S.typhimurium | Blue agglutinate, orange background |
| 4. | Salmonella Serogroup C eg. S.newport | Green agglutinate, purple background |

(b) Antigen detection in biological fluids.

Spinal fluid taken from a patient with meningitis was tested, as described above, against the Salmonella polyvalent latex. Agglutination of the red latex particles occurred indicating the presence of antigen from Salmonella serogroup A organisms in the spinal fluid. Spinal fluid from an uninfected person did not cause agglutination of the latex.

**Claims**

1.  A method of testing for the presence of a ligand in an aqueous liquid medium comprising the steps of:
    A. mixing a sample of the said medium with a reagent which comprises
    particles of a first colour having attached thereto antibody bindable to the said ligand, in admixture with particles of a second colour having attached thereto immunoglobulin from a non-inoculated animal,
    whereby non-specific interaction between the said sample and the said reagent is indicated by agglutination of both the said particles of the first colour and the said particles of the second colour and the presence of the said ligand in the said sample, free from said non-specific interaction, is indicated by agglutination of the said particles of the first colour alone;
    B. observing whether or not agglutination occurs; and
    C. determining the colour of the agglutinate.

2.  The method of claim 1 wherein the antibody was raised in a rabbit.

3.  The method of either of claims 1 and 2 wherein the said particles are of polystyrene latex.

4.  The method of any of claims 1 to 3 wherein the ligand is a bacterial or viral antigen.

5.  The method of claim 4 wherein the ligand is an antigen characteristic of Haemophilus, Neisseria or Streptococcus organisms.

6.  The method of claim 4 wherein the ligand is an antigen characteristic of Streptococcus organisms of serogroup A.

7.  The method of claim 4 wherein the ligand is an antigen characteristic of Salmonella organisms.

8.  A test reagent for the presence of a ligand in a sample of an aqueous liquid medium, said reagent comprising
    particles of a first colour having attached thereto antibody bindable to the said ligand, in admixture with particles of a second colour having attached thereto immunoglobulin from a non-inoculated animal,
    whereby non-specific interaction between the said sample and the said reagent is indicated by agglutination of both the said particles of the first colour and the said particles of the second colour and the presence of the said ligand in the said sample, free from said non-specific interaction, is indicated by agglutination of the said particles of the first colour alone.

9. The reagent of claim 8 wherein the antibody was raised in a rabbit.

10. The reagent of either of claims 8 and 9 wherein the said particles are of polystyrene latex.

11. The reagent of any of claims 8 to 10 wherein the ligand is a bacterial or viral antigen.

12. The reagent of claim 11 wherein the ligand is an antigen characteristic of Haemophilus, Neisseria or Streptococcus organisms.

13. The reagent of claim 11 wherein the ligand is an antigen characteristic of Streptococcus organisms of serogroup A.

14. The reagent of claim 11 wherein the ligand is an antigen characteristic of Salmonella organisms.

**Revendications**

1. Procédé pour tester la présence d'un ligand dans un milieu liquide aqueux, comprenant les stades :
   A. de mélange d'un échantillon de ce milieu avec un réactant qui comprend des particules d'une première couleur auxquelles est attaché un anticorps fixable par le ligand, en mélange avec des particules d'une deuxième couleur auxquelles est attachée une immunoglobuline provenant d'un animal non inoculé, de façon que l'interaction non spécifique entre l'échantillon et le réactant soit indiquée par l'agglutination tant des particules de la première couleur que des particules de la deuxième couleur et que la présence du ligand dans l'échantillon, sans l'interaction non spécifique, soit indiquée par l'agglutination des particules de la première couleur uniquement;
   B. d'observation si l'agglutination a lieu ou non, et
   C. de détermination de la couleur du produit d'agglutination.

2. Procédé suivant la revendication 1, dans lequel l'anticorps a été formé chez un lapin.

3. Procédé suivant la revendication 1 ou 2, dans lequel les particules sont celles d'un latex de polystyrène.

4. Procédé suivant l'une quelconque des revendications 1 à 3, dans lequel le ligand est un antigène bactérien ou viral.

5. Procédé suivant la revendication 4, dans lequel le ligand est un antigène caractéristique d'organismes Haemophilus, Neisseria ou Streptococcus.

6. Procédé suivant la revendication 4, dans lequel le ligand est un antigène caractéristique d'organismes Streptococcus du sérogroupe A.

7. Procédé suivant la revendication 4, dans lequel le ligand est un antigène caractéristique d'organismes Salmonella.

8. Réactant pour tester la présence d'un ligand dans un échantillon d'un milieu liquide aqueux, lequel réactant comprend des particules d'une première couleur auxquelles est attaché un anticorps fixable par le ligand, en mélange avec des particules d'une deuxième couleur auxquelles est attachée une immunoglobuline provenant d'un animal non inoculé, de façon que l'interaction non spécifique entre l'échantillon et le réactant soit indiquée par l'agglutination tant des particules de la première couleur que des particules de la deuxième couleur et que la présence du ligand dans l'échantillon, sans l'interaction non spécifique, soit indiquée par l'agglutination des particules de la première couleur uniquement.

9. Réactant suivant la revendication 8, dans lequel l'anticorps a été formé chez un lapin.

EP 0 174 195 B1

**10.** Réactant suivant la revendication 8 ou 9, dans lequel les particules sont celles d'un latex de polystyrène.

**11.** Réactant suivant l'une quelconque des revendications 8 à 10, dans lequel le ligand est un antigène bactérien ou viral.

**12.** Réactant suivant la revendication 11, dans lequel le ligand est un antigène caractéristique d'organismes Haemophilus, Neisseria ou Streptococcus.

**13.** Réactant suivant la revendication 11, dans lequel le ligand est un antigène caractéristique d'organismes Streptococcus du sérogroupe A.

**14.** Réactant suivant la revendication 11, dans lequel le ligand est un antigène caractéristique d'organismes Salmonella.

**Patentansprüche**

**1.** Testverfahren auf die Anwesenheit eines Liganden in einem wässerigen flüssigen Medium, das die folgenden Schritte umfaßt:
A. Mischen einer Probe des Medium mit einem Reagens, das Teilchen von einer ersten Farbe, an denen ein an den Liganden bindungsfähiger Antikörper haftet, in Mischung mit Teilchen von einer zweiten Farbe, an denen Immunoglobulin von einem nicht-angeimpften Lebewesen haftet, wobei nicht-spezifische Wechselwirkung zwischen der Probe und dem Reagens durch Agglutination sowohl der Teilchen von der ersten Farbe als auch der Teilchen von der zweiten Farbe angezeigt wird und die Anwesenheit des Liganden in der Probe, frei von der nicht-spezifischen Wechselwirkung, durch Agglutination der Teilchen von der ersten Farbe allein angezeigt wird,
B. Feststellen, ob Agglutination erfolgt oder nicht, und
C. Bestimmen der Farbe des Agglutinats.

**2.** Verfahren nach Anspruch 1, in dem der Antikörper in einem Kaninchen gebildet wurde.

**3.** Verfahren nach einem der Ansprüche 1 und 2, in dem die Teilchen aus Polystyrollatex bestehen.

**4.** Verfahren nach einem der Ansprüche 1 bis 3, in dem der Ligand ein bakterielles oder virales Antigen ist.

**5.** Verfahren nach Anspruch 4, in dem der Ligand ein für Haemophilus-, Neisseria- oder Streptococcusorganismen charakteristisches Antigen ist.

**6.** Verfahren nach Anspruch 4, in dem der Ligand ein für Streptococcusorganismen der Serogruppe A charakteristisches Antigen ist.

**7.** Verfahren nach Anspruch 4, in dem der Ligand ein für Salmonellaorganismen charakteristisches Antigen ist.

**8.** Testreagens auf die Anwesenheit eines Liganden in einer Probe eines wässerigen flüssigen Mediums, welches Reagens Teilchen von einer ersten Farbe, an denen ein an den Liganden bindungsfähiger Antikörper haftet, in Mischung mit Teilchen von einer zweiten Farbe, an denen Immunoglobulin von einem nicht-angeimpften Lebewesen haftet, umfaßt, wobei nicht-spezifische Wechselwirkung zwischen der Probe und dem Reagens durch Agglutination sowohl der Teilchen von der ersten Farbe als auch der Teilchen von der zweiten Farbe angezeigt wird und die Anwesenheit des Liganden in der Probe, frei von der nicht-spezifischen Wechselwirkung, durch Agglutination der Teilchen von der ersten Farbe allein angezeigt wird.

**9.** Reagens nach Anspruch 8, wobei der Antikörper in einem Kaninchen gebildet wurde.

**10.** Reagens nach einem der Ansprüche 8 und 9, wobei die Teilchen aus Polystyrollatex bestehen.

7

**11.** Reagens nach einem der Ansprüche 8 bis 10, wobei der Ligand ein bakterielles oder virales Antigen ist.

**12.** Reagens nach Anspruch 11, in dem der Ligand ein für Haemophilus-, Neisseria- oder Streptococcusorganismen charakteristisches Antigen ist.

**13.** Reagens nach Anspruch 11, in dem der Ligand ein für Streptococcusorganismen der Serogruppe A charakteristisches Antigen ist.

**14.** Reagens nach Anspruch 11, in dem der Ligand ein für Salmonellaorganismen charakteristisches Antigen ist.